# EUROPEAN PATENT APPLICATION

(11) **EP 4 696 298 A2**
(43) Date of publication of application: **18.02.2026**
(21) Application number: 25221610.6
(22) Date of filing: 04.11.2023
(51) Int. Cl.: A61K 8/23

(54) **PERSONAL CLEANSING COMPOSITION**

(30) Priority: 04.11.2022 ZA 202212072
(62) Divisional of application: 23820995.1
(71) Applicant: Byrne, Daniel, Rathconrath Mullingar (IE)
(72) Inventor: Byrne, Daniel, Rathconrath Mullingar (IE)

(57) **Abstract**

A personal cleansing composition having comprising a physiologically active amount of carnosine, wherein the personal cleansing composition is in the form of a shower gel, bubble bath, soap, or water-soluble delivery agent. The personal cleansing composition further comprises physiologically active ingredients including magnesium salt and caffeine in an amount of approximately 0.1 % to 1.0 % by weight of the total composition. The carnosine and magnesium salt form a carnosine-magnesium complex on activation with water, and wherein the personal cleansing composition further comprises transdermal absorption enhancers for the delivery of the physiologically active ingredients selected from the group consisting of salicylic acid in an amount of approximately 0.5 % to 2.0 % by weight of the total composition and azone oil in an amount of approximately 0.25 % to 1 % by weight of the total composition.

## Description

### Cross-Reference To Related Application

This divisional patent application derives benefits of EP regional phase patent application no. 23820995.1 filed on June 4, 2025, from international patent application no. PCT/IB2023/061135 which derives priority benefit of application no. 2022/12072 entitled "Personal Cleansing Composition" and filed on November 4, 2022.

### Background of the Invention

The present invention relates to a personal cleansing composition, particularly but not exclusively to a shower gel, bath salt, bath bomb, bath melt, or bar of soap.

It is common for users having exercised to want to shower or bathe in order to remove sweat and dirt from their bodies, but also to have a relaxing experience following physical exertion. Many compositions are available which can assist with regeneration or restoration of the body of an exercised individual. However, where topically applicable ingredients are provided, these tend to be applied as a cream, ointment or tincture, which is dispersed and/or washed off during the cleansing process. This requires re-application of the cream to achieve maximum efficacy following washing, which can be wasteful, and can also limit the regeneration process if the user has long since finished exercising by the point of re-application.

### Brief Summary of Embodiments of the Invention

The present invention seeks to provide a means of providing improved penetration of physiologically active ingredients which would otherwise not be able to remain on the skin for sufficient durations during a bath or shower experience.

According to a first aspect of the invention, there is provided a personal cleansing composition comprising:
(i) a physiologically active amount of carnosine;
(ii) a physiologically active amount of salicylic acid, preferably in an amount of between about 0.5% to 2.0% concentration; and
(iii) between about 0.25 % to 1% azone oil, or laurocapram oil,

wherein the salicylic acid and azone oil act synergistically as a penetration enhancer that increases the skin's permeability for transdermal absorption of the physiologically active ingredients, and
wherein the personal cleansing composition is in the form of a gel, soap, or water-soluble delivery agent.

### Detailed Description of the Embodiments of the Invention

Carnosine advantageously increases the formation of collagen, is an anti-oxidant, and is generally extremely beneficial to a user's skin, particularly if applied topically. One of the most appropriate access points to a user's skin is during a washing or cleansing experience, and therefore the provision of the composition which is suitable for use in such an environment improves the user's ability to maintain a healthy skin appearance.

Preferably, the carnosine may be present in an amount of approximately 0.1 % to 1.0 %, including any subrange therein, by weight of the total composition, and more preferably present in an amount of 0.2 % by weight of the total composition.

The provision of approximately of 0.1 % to 1.0 %, more preferably 0.2 % by weight of the carnosine in the total composition advantageously provides the physiological benefits of the carnosine without adversely affecting the consistency or aroma of the personal cleansing composition.

Salicylic acid acts as a penetration enhancer that increases the skin's permeability for transdermal absorption of active ingredients. Typically, the salicylic acid may be present in an amount of approximately 0.50 % to 2.00 %, including any subrange therein, by weight of the total composition.

Azone oil, also known as laurocapram oil is an oil-soluble carrier for oil-soluble active ingredients. It increases the skin penetration of both oil-soluble actives and water-soluble actives. It enhances water-soluble active by increasing the fluidity of cell membranes and reducing diffusion resistance. It is a safe penetration enhancer that does not irritate the skin. It offers good delivery and assists with the penetration of functional additives into the skin. It further boosts stability of active ingredients without skin stimulation.

Typically the azone oil may be present in an amount of approximately 0.25 % to 1.00 % including any subrange therein, by weight of the total composition.

It has been determined that azone oil acts synergistically with salicylic acid as a permeation enhancer (Zhao et al., 2016), but the applicant is the first to show that this combination when used in the personal cleansing composition of the invention is effective to improve penetration of physiologically active ingredients in the composition which would otherwise not be able to remain on the skin for sufficient durations during a bath or shower experience.

Optionally, the personal cleansing composition may further comprise a magnesium salt. Magnesium has a relaxing effect on the body and can serve to reduce inflammation. Prolonged exposure to magnesium on the skin allows absorption thereof which can assist with restoration of the body, particularly after exercise. On activation with water the carnosine and magnesium salt may form a carnosine magnesium complex.

Such a complex may improve magnesium uptake through the skin, which improves the effectiveness of the associated anti-inflammatory capabilities of the composition. Preferably, the magnesium salt may be present in an amount of approximately 0.5 % to 2.0 %, including any subrange therein, by weight of the total composition. More preferably, the magnesium salt may be present in an amount of 1.0 % by weight of the total composition. In any event, it is preferred that the magnesium salt may be present in a larger amount by weight of the total composition that the carnosine.

The increased absorption of the magnesium salt through the skin of the user by combination with the carnosine, the salicylic acid and the azone oil is one of the main advantages of the present composition, and the above-referenced ranges have been found to have a most efficacious improved effect on the absorption properties thereof.

In preferred embodiments, the personal cleaning composition may further comprise caffeine.

Caffeine, when used topically, can soothe skin after periods of intense activity, and can also result in a firmer appearance to the skin.

Optionally, the caffeine may be present in an amount of between approximately 0.1 % to 1.0% including any subrange therein, by weight of the total composition, and more preferably the caffeine may be present in an amount of 0.2 % by weight of the total composition. The efficacious effects of the caffeine can be realised with relatively small percentages by weight, resulting in a firmer appearance to the user's skin, which, when coupled with the benefits of the carnosine, and the penetration enhancing activity of the salicylic acid and the azone oil, leads to an improved user appearance.

At least one essential oil may be provided as part of the composition, and which is selected from the set of: camphor oil; eucalyptus oil; clove bud oil; and almond oil.

Essential oils can not only add vibrant aromatic sensations to the composition but may also have advantageous properties which may further assist with the recovery or revitalisation of the user.

Preferably, the at least one essential oil may be present in an amount of between approximately 0.1 % to 2.0 % including any subrange therein, by weight of the total composition, and more preferably, the at least one essential oil is present in an amount of 1.0% by weight of the total composition.

It is preferred that greater amounts of essential oils may be utilised when compared with the other ingredients, since the essential oils may impart beneficial fragrances or aromas which are significant for the final composition.

The personal cleaning composition may optionally further comprise arnica extract.

Arnica extract has significant anti-bruising capabilities, reducing inflammation around a bruised area. This may be of particular importance where a personal cleansing composition is provided which is suitable for use after exercise.

The arnica extract may be present in an amount of between approximately 0.1 % to 0.5 %, including any subrange therein, by weight of the total composition, and more preferably, the arnica extract may be present in an amount of 0.4 % by weight of the total composition.

Relatively small concentrations of arnica extract can result in improvements to the reductions in the appearance of bruising, further improving the benefits of the present composition for the appearance of the user's skin.

An exfoliant may also be provided as part of the personal cleaning composition.

Exfoliation of the user's skin using the composition can assist with the removal of dirt, sweat and dead skin, and may further improve the ability of absorption of the active ingredients including magnesium, for example, through the skin.

In one embodiment, the exfoliant may be present in an amount of between approximately 0.1 % to 0.5 % including any subrange therein, by weight of the total composition, and more preferably, in an amount of 0.2 %. It is preferred that the total amount of exfoliant be limited, to prevent over-exfoliation of the user's skin during a cleansing experience, which would otherwise be irritating or painful.

Preferably, the composition may be in the form of an aqueous gel, such as a shower gel or bubble bath gel.

A shower gel or bubble bath provides a mechanism by which the user can topically apply the physiologically active component of the composition to the skin, so as to improve in particular magnesium absorption, in contexts which would otherwise not normally be associated with prolonged skin contact and therefore efficacy.

Alternatively, the composition may be in the form of a water-soluble delivery agent formed as a bath salt. In another embodiment, the composition may be in the form of a water-soluble delivery agent formed as a bath bomb or bath melt.

Such arrangements allow for a bath to be run and the composition inserted in such a way as to allow for the ingredients, and in particular the active ingredients carnosine and magnesium, to be maintained within the bath water for prolonged exposure to the user's skin, with enhanced penetration into the skin through the inclusion of the salicylic acid and azone oil.

In a further embodiment, the composition may be in the form of a bar of soap. A soap bar is another effective agent for delivery of the physiologically active ingredients to a user's skin during a cleansing experience.

Non-limiting examples of the present invention will now be provided.

The personal cleansing composition may be formed as a shower gel, suitable for use by a user washing themselves in a shower and/or shower cubicle, though the product could evidently be used in any context in which a user washes themselves. Shower gel is an aqueous composition, typically formed as an emulsion of water and a detergent.

Fragrance may be added, in addition to one or more additional skin-cleansing agents.

One exemplary embodiment may comprise ingredients including water and non-ionic surfactants with good dermatological compatibility and/or viscosity-enhancing effects, such as those containing fatty alcohol polyglycosides, and in particular, C8-C16 or C12-C16 fatty alcohol polyglycosides. Other surfactants may be included, such as anionic surfactants like sodium laureth sulfate, or co-surfactants such as cocamidopropyl betaine.

Other ingredients may include xantham gum extracts, or similar products to improve the flow properties of the shower gel, preservatives such as sodium chloride, sodium benzoate, or mixed preservatives, for example, based upon dehydroacetic acid and benzyl alcohol. Colourings or perfumes may also be included, examples of which may include menthol, for improving the revitalisation of a user immediately after exercise, lemon fragrance, having restorative properties, and/or antimicrobial agents for full body cleansing.

Active ingredients which are designed to improve the revival, restoration or relaxation properties of the shower gel when used can also be included.

In particular, carnosine, specifically L-carnosine or P-alanyl-L-histidine, is included. Typically, carnosine is utilised by the body, and is ingested orally. However, it has been found that advantageous effects can be achieved for the body when applied topically, and particularly during a washing or cleansing environment. Carnosine increases the formation of collagen in the body and is an anti-oxidant to offer protection against free radical or glycation damage.

The provision of carnosine which is applied topically can assist with a variety of skin complaints. However, whereas carnosine has previously been incorporated into creams or similar emollients, it has been found that advantageous effects for the skin can still be achieved if the carnosine is provided in a water-soluble or -applicable delivery agent. The application of a shower gel containing carnosine can provided these benefits, whilst providing an invigorating shower experience for the user.

In order to be physiologically effective, it is preferred that the carnosine be present in an amount of between approximately 0.1 % to 1.0 % by weight of the total composition, and in one preferred embodiment of the shower gel, may be present in an amount of 0.2 % by weight of the total composition. The total amount may be dependent upon the desirable characteristics of the shower gel, and how carnosine may otherwise affect said characteristics, for example, viscosity or aroma.

In order to improve the penetration of active compounds including the carnosine into the skin during use of the composition, the applicant has found that salicylic acid in combination with azone oil acts synergistically as a penetration enhancer that increases the skin's permeability for transdermal absorption of the active ingredients in the skin cleansing composition on the invention. Typically, the salicylic acid may be present in an amount of approximately 0.5% to 2.0 % by weight of the total composition, more preferably and the azone oil is present in an amount of up to approximately between 0.1 % to 1.0 % by weight of the total composition, more preferably.

Carnosine can also improve the ability of the skin to absorb magnesium, which can assist with inflammation of, in particular, the user's muscles. As such, the shower gel may comprise a magnesium salt, such as magnesium sulfate, and the provision of the carnosine results in improved uptake of the magnesium through the skin during and potentially after the shower experience. The improved absorption capability may be at least in part achieved by the chelation of the magnesium ion with the carnosine, forming a more efficacious and physiologically active composition, such as a carnosinemagnesium complex, preferably on activation with water.

In order to achieve a desirable effect from the magnesium, it is preferred that it is present in the composition in an amount of 0.5 % to 2.0 % by weight of the total composition, and in the example where the shower gel comprises 0.2 % by weight of carnosine, there is 1.0 % by weight of magnesium sulfate. In any event, it is preferred that the magnesium salt be present in an amount which is greater than that of the carnosine, in order for the efficacy of the composition to be maintained, and may be present of the order of several times the amount, for example two, three, four or five times the amount of magnesium salt with respect to the carnosine.

Other efficacious compounds for restoration of the skin could also be considered. For example, caffeine may be introduced, which acts to soothe skin after a period of intense activity, providing a firming effect. Efficacious amounts of caffeine have been found to be an amount of between about 0.1 % to 1.0 % by weight of the total composition, and in the above -referenced shower gel, the caffeine is present in an amount of 0.2 % by weight of the total composition. Arnica extract could also be considered, since it has effective anti-inflammatory capabilities, derivable from its primary pharmacologically active constituent, helenalin, which has both anti-inflammatory and analgesic properties, particularly when applied topically. The arnica extract is present in an amount of between about 0.1 % to 0.5 % by weight of the total composition, and preferably in an amount of 0.4 %, as is the case in the above -referenced shower gel.

Essential oils could also be included in the shower gel, which assist with increasing the flow of blood to a particular area of the body, thereby improving the body's natural response to reduce pain and swelling, and this may be particularly important as part of a post-exercise routine. Camphor oil, for example, produces a warming sensation when applied vigorously, or a cooling sensation if applied more gently to the skin, via activation of ion channels in the skin via camphor in the camphor oil. Other essential oils could be considered and are discussed in detail in respect of the later embodiments below.

The at least one essential oil is present in an amount of between about 0.1 % to 2.0 % by weight of the total composition, and this may be applicable for the total amount by weight of all essential oils, or for the or each individual essential oil where more than one is present. Essential oil is present in an amount of 1.0 % by weight of the total composition for the above -referenced shower gel.

An exfoliant may also be considered, which assists with exfoliation of the skin of the user during application of the shower gel. One appropriate exfoliant might be Luffa Cylindrica, which is a naturally derived mild abrasive ingredient, but other mildly abrasive materials could also be provided in suspension in the shower gel. Exfoliant, such as Luffa Cylindrica, may preferably be present in an amount of between about 0.1 % to 0.5 % by weight of the total composition, and is present in an amount of 0.2 % by weight of the total composition for the above -referenced shower gel.

Such a shower gel is able to provide a user with an invigorating shower experience and may be tailored accordingly to the type of experience the user requires. For instance, a reviving shower experience may be implemented by the inclusion of menthol, mint, or tea tree oil to improve the aromatics of the experience, assisting with clearing of the user's air passages.

Alternatively, a restorative experience may be created by the provision of citrus fragrance, in particular lemon fragrance, which provides a zesty aroma which energises the user. Relaxation effects could also be achieved, for example by the provision of calming aromas such as lavender, rose, or similar floral fragrances.

A bubble bath, which is also formed as an aqueous gel, may also be provided having similar or identical compositions to the shower gel. Typically, a bubble bath would comprise ingredients designed to reduce the viscosity of the gel with respect to a shower gel, but otherwise, the same kind of ingredients will be equally applicable.

It will be appreciated that water-soluble delivery agents could be used to impart the restorative properties of the carnosine and other physiologically active components of the shower gel. Similar or identical percentages by weight of the relevant components to those referenced in respect of the shower gel will also be applicable in respect of the water-soluble delivery agents.

For instance, various arrangements may be suitable for inclusion as part of a bathing experience. Bath salts may be one such delivery mechanism, typically formed as water-soluble, pulverised minerals which can be dissolved into a bath to improve the bathers experience.

Dead sea salt may form the basis of any bath salt mixture, as could Epsom salts, which naturally contain magnesium sulfate, in addition to other salts. Other ingredients may include emollients, humectants, or lubricants.

In such an arrangement, it may be useful to include one or more essential oils to improve the experience, such as clove bud or eucalyptus oil, which respectively have antiseptic and anti-inflammatory properties The salt mixture could be treated so as to include any or all of the carnosine, magnesium sulfate and/or caffeine, in particular, in order to create the same benefits as are experienced with the shower gel.

Bath bombs are another alternative delivery mechanism, typically formed as spherical or similarly shaped items which have material products designed to effervesce in bathwater. Generally, a bath bomb with comprise a weak acid, such as citric acid, and a bicarbonate base, such as sodium bicarbonate, which are activated upon contact with water to provide the effervescence associated with bath bombs, and additional preservatives or stabilisers may also be included. The remaining ingredients may then be added in with the dry, hard-packed mixture which forms the bath bomb body.

Menthol may be an appropriate ingredient to utilise in such a bath bomb, since there is a synergistic effect with the effervescence of the bath bomb to release aromas. Essential oils, in particular clove bud and eucalyptus oils, are preferably included with the mixture prior to drying, along with any carnosine, caffeine and/or magnesium sulfate. A similar arrangement may be created with bath melts, which are designed to disperse within hot water. It is therefore similar to a bath bomb, but the dispersal of the ingredients is dependent upon thermal degradation of the structure of the bath melt, rather than being as a result of water-activation of a chemical reaction. Typically, the bath melt is formed from a material having a low melting temperature, such as a fat compound or vegetable or nut butter.

The bath melt may melt and thereby created a layer of oil in the bath, but also may be viably used as a scrub before complete melting occurs. As such, exfoliant may be a suitable additive to a bath melt, in addition to essential oils, particularly eucalyptus and camphor oil, arnica extract, magnesium salt, carnosine, salicylic acid and azone oil. A bath melt could also be created by providing these active ingredients encased in a water-soluble package, for example, from a soluble polyvinyl alcohol package. Such packaging may dissolve over time, allowing for a staged release of the physiologically active components. This may allow additional constituents to be included within the packaging, which may not otherwise be suitable for, for example, bath bombs or unpackaged melts. This may include, for instance, improved lathering or chelating agents, such as pentasodium pentetate, or tetrasodium etidrononate, which may alter the amount of bubbles or foam produced.

It may also be possible to provide a bar of soap having the same physiologically active ingredients, which allows the user to apply the relevant ingredients topically by using the bar of soap. Other than carnosine, salicylic acid and azone oil, essential oils may be readily added to the soap, providing the advantages described above. Additional constituents may include almond or shea butter, or similar moisturisers, which assist with moisturisation of the skin. Exfoliants can be added, and oatmeal could be considered as an alternative to Luffa Cylindrica, for example. Other components, flavourings or scents could also be considered in specific bars of soap, such as eucalyptus and clove, aloe vera for antiinflammatory purposes, and black pepper and patchouli in which the black pepper also acts as an exfoliant, by way of example only.

Again, similar or identical percentages by weight of the relevant components to those referenced in respect of the shower gel will also be applicable in respect of the soap.

It is therefore possible to provide a personal cleansing composition which allows for the application of carnosine or similar physiologically active agents to be applied to a user with enhanced penetration by inclusion of a combination of salicylic acid and azone oil, in an aqueous environment, such as a bath or shower. This allows the benefit of the ingredients to the skin to be effective without the danger of being washed off as would be the case for a topically applied cream or emollient, for instance.

There are many transdermal delivery systems available including DMSO patches and other chemical delivery systems. Some are more effective than others. There is limited evidence but recent discoveries indicate certain delivery methods like salicylic acid for the transdermal transportation of carnosine to musculoskeletal tissue can have therapeutic effects. Many studies include clinical trials on humans and animals with magnetic resonance imaging for gauging effective absorption. In the absence of human or animal skin, a synthetic skin can be used in conjunction with a Franz Diffusion Cell (FDC). An FDC is usually used on excised skin but in the absence of skin, a semipermeable membrane that mimics the effects of transdermal absorption can be used. Polymethylsiloxane (PDMS) is one example of a synthetic membrane that is used quite often to simulate the skin because it is both hydrophobic and possesses rate-limiting properties like real skin. The synthetic membrane should have minimum diffusion resistance, act as a continuous medium for dermal transfer, and have a porous quality similar to that of human skin. In the United States, the Federal Drug Administration (FDA) has suggested that simple, porous synthetic membranes are suitable for assessing topical formulation performance since they act as a support yet are not rate-limiting barriers.

### Experimental section

A comparative study was performed on different membranes used in a Franz Diffusion Cell.

Companies that prepare these membranes in both the US and UK were used for comparison. Table 1 shows both cellulose and polymer membranes employed in Franz diffusion cells experiments in this study. The membrane thickness, molecular-weight cut off (MWCO) range, nominal pore size, porosity and individual manufacturers are included. Note that all membranes listed are porous because this study focused only on membranes for quality control purposes. PDMS (non-porous) was listed as a baseline for performance comparison.

**Table 1**

| All values are provided by manufacturers (p - membrane porosity, r - membrane tortuosity). | | | | | | | | |
|---|---|---|---|---|---|---|---|---|
| **Membrane** | **Poly mer** | **MWCO (kDa)** | **Pore size (µm)** | **Thickness(µm)** | **ρ (%)** | **τ** | **Source** | **Batch no.** |
| ***cellulose based*** | | | | | | | | |
| Visking | RC | 12-14 | - | 20b | - | - | Medicell (London, UK) | DTV120 00.05.00 0 |
| Cuprophan | RC | 10 | - | 10b | - | - | Medicell (London, UK) | N/A |
| Benzoylated tubing | RC | 1.2-2 | - | 35b | - | - | Sigma (Dorset, UK) | 074K701 2 |
| Cellulose ester | CE | 0.5 | - | 80b | - | - | Spectrum lab (Californ ia) | 131060 |
| Cellulose nitrate | CN | | 0.45 | 125 | 66-84 | - | Whatma n (UK) | N/A |

| ***polymer based*** | | | | | | | | |
|---|---|---|---|---|---|---|---|---|
| AN 69 | PAN | 40 | - | 25b | - | - | Hospal (Hunting don, UK) | N/A |
| Biodyne | PA | na | 0.45 | 152 | 50- | - | Pall (Portsmo uth, UK) | b- 50046, c-189051 |
| Supor | PES | na | 0.45 | 145 | 75 | ~1-1.5 | Pall (Portsmo uth, UK) | 55083 |
| Tuffryn | PS | na | 0.45 | 145 | 80 | ~1-1.5 | Pall (Portsmo uth, UK) | 60669 |
| Nuclepore | PC | na | 0.1 | 10 | 60 | ~1 | Whatma n (New Jersey, USA) | 6018023 |
| Cyclopore | PC | na | 0.1 | 10 | 8 | ~1 | Whatma n (New Jersey, USA) | 060.0131 /6E8/L-3-L |
| Celgard 3500 | PP | na | 0.05 | 20 | 4 | - | Hoechst (New Jersey, USA) | 293485 |
| Silicone | PDM S | na | - | 400 | 35-48 | - | SAMCO (Nuneato n, UK) | 19T0.3-1000-60M1 |

| | | | | | | | | |
|---|---|---|---|---|---|---|---|---|
| RC - Regenerated cellulose, CE - Cellulose esters, CN - Cellulose nitrate, PAN - Polyacrylonitrile, PA - Polyamide (nylon), PES - Polyethersulfone, PS - Polysulfone, PC - Polycarbonate, PP -Polypropylene, PDMS - Polydimethylsiloxane, b dry thickness measured using a digital micrometer (Mitutoyo, UK). | | | | | | | | |

### Franz Diffusion Cell

Transdermal delivery efficiency was performed on a custom built Franz Diffusion Cell. The diffusion area of the Franz cells was 59.6 ± 3.1 mm2 and receptor volume was 11.7 + 0.1 mL.

### Delivery System/Summary of the Invention

At higher exercise intensities, the rate of hydrogen ion production lessens. Highly reactive hydrogen ions cause a fall in pH in the muscles, a process referred to as acidification. Carnosine reduces this effect on musculoskeletal tissue. Due to the fact that carnosine breaks down into beta-alanine and histidine in the bloodstream after absorption, ingesting it directly is not effective.

Hence why we are proposing a topical gel mixture of carnosine at a weight percentage of around 0.1% to 1.0% (preferably around 0.2% of the total weight), with salicylic acid in concentrations between 0.5% to 2.0%, and Azone oil (Laurocapram) at 0.25% to 1.0% composition. Salicylic acid has been well studied as an absorption enhancer. Azone oil enhances the permeation of both hydrophilic and lipophilic molecules, while also moisturizing the skin.

An active ingredient that is topically applied to the skin must be able to penetrate the uppermost barrier of the skin, the Stratum Corneum (SC), before being delivered to the active site in the required concentrations. However, due to the lipophilic properties of the SC, hydrophilic molecules are not able to penetrate the Stratum Corneum effectively. Therefore, obtaining skin bioavailability of hydrophilic active substances, like peptides or peptide-like substances is challenging.

The topical composition may be in the form of a gel, a cream, a solution, an ointment, a paste, a lotion, a patch, a spray or a foam. The preferred composition is in the form of a gell. The topical additives may comprise a lubricant, a carrier, a thickening agent, a preservative, a surfactant, a moisturizing and an emollient agent. It may also comprise a warming agent. Salicylic acid functions as an endogenous regulator of heat production.

Specifically, the present disclosure relates to a topical composition comprising a physiologically active amount of a carnosine-azone oil-salicylic acid complex that is able to penetrate the dermal layer into musculoskeletal tissue. The composition of the current disclosure may also be used to increase the athletic performance and recovery of a subject.

When the topical composition comprising a carnosine, salicylic acid, azone oil mixture is applied to the skin, after 24 hours between 0.0033% and 0.02% of the carnosine applied to the skin may pass the skin layer of a EpiDerm^{™} reconstructed human epidermis model and may be found in the medium.

When the topical composition comprising a carnosine, salicylic acid, azone oil mixture is applied to the skin, after 48 hours between 0.005% and 0.01% of the carnosine applied to the skin may pass the skin layer of an EpiDerm^{™} reconstructed human epidermis model and may be found in the medium.

When the topical composition comprising a carnosine, salicylic acid, azone oil mixture is applied to the skin, after 48 hours between 0.05% and 0.07% of the carnosine applied to the skin may be found in the homogenates of the skin model of EpiDerm^{™} reconstructed human epidermis model. The multiple layers of the topical composition may be applied to an area of skin.

One or more topical applications may be performed on the skin from between 1 to 5 times a day, but preferably between 1 to 3 times a day. The topical gel may be applied to the skin at any time of day and before, after, or during exercise. Preferably the topical gel will be applied to the skin pre and post exercise.

### Experimental Section Materials and Methods

Evaluation of the transepidermal penetration of the carnosine composition in gel formulation was performed on 3D-skin models.

The focus of the described experiments is to investigate the transdermal delivery of L-carnosine to human skin by the gel formulation containing L-carnosine in association with salicylic acid and azone oil, with the primary goal of improving bioavailability.

EpiDerm^{™} Reconstructed Human Epidermis (RHE) model consists of a 3-D printed epidermal tissue grown at the air liquid interface from normal human keratinocytes. The model is histologically similar to human epidermis, having all differentiated cellular layers, and a functional permeability barrier.

Before carrying out the treatments, the medium was withdrawn and fresh medium was added. For the treatments, 10 microliters of the three separate mixtures (A: carnosine-complex; B: free carnosine without the enhancer; C: salicylic acid and azone delivery) were each topically applied over separate RHE tissues either in a single dose for 24 h or in a repeated application every 24 h for a total of 48 h. The control tissue was only exposed to the medium. Since no differences between doses applied at 24 and 48 h were observed for the control tissues, the samples were pooled and presented as the control group. All mixtures and control samples were assayed in triplicate by using three sets of RHE for each group.

Carnosine, also known as beta-alanyl-L-histidine, has shown evidence of improving muscle recovery. Hence why it is being explored as a topical ointment with the assistance of salicylic acid and Azone Oil (Laurocapram) as a means of effective transdermal delivery. Other means of Carnosine delivery have involved ingestible supplements, which is what makes this delivery system unique. Glycolysis is a process that the human body undergoes a metabolic process that converts glucose into pyruvate, and in most organisms, occurs in the liquid part of cells, the cytosol. As the intensity of a workout increases, comparable amounts of lactate and hydrogen ions become the products of glucose metabolism. Carnosine is an anti-glycolating agent that reduces the amount of glycolated byproducts. The combination of carnosine, salicylic acid, and azone oil are believed to combat these effects. Carnosine is also believed to reduce the Hayflick phenomenon. This is believed to limit the number of times a normal somatic, differentiated human cell population will divide before cell division stops. Whenever a cell undergoes mitosis (cell division), the telomeres on the ends of each chromosome shorten slightly. Cell division will cease once telomeres shorten to a critical length
The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention as defined herein.

### References

Zhao, Q., Dai, C., Fan, S. et al. Synergistic efficacy of salicylic acid with a penetration enhancer on human skin monitored by OCT and diffuse reflectance spectroscopy. Sci Rep 6, 34954 (2016). https://doi.org/10.1038/srep34954

## Claims

1. A personal cleansing composition, the composition comprising:
physiologically active ingredients including carnosine or functional analogue thereof selected from N-acetyl-carnosine, anserine, balenine, ophthalmic carnosine derivatives and pharmaceutically acceptable salts thereof;
a physiological active amount of salicylic acid in an amount of approximately 0.5 % to 2.0 % by weight of the total composition and azone oil in an amount of approximately 0.25 % to 1 % by weight of the total composition;
wherein the salicylic acid and the azone oil act synergistically as a penetration enhancer to increase skin's permeability of a user for transdermal absorption of the physiologically active ingredients.

2. A personal cleansing composition, the composition comprising:
At least a physiologically active ingredient;
a physiological active amount of salicylic acid in an amount of approximately 0.5 % to 2.0 % by weight of the total composition and azone oil in an amount of approximately 0.25 % to 1 % by weight of the total composition;
wherein the salicylic acid and the azone oil act synergistically as a penetration enhancer to increase skin's permeability of a user for transdermal absorption of the physiologically active ingredients.

3. A personal cleansing composition comprising:
a physiological active amount of salicylic acid in an amount of approximately 0.5 % to 2.0 % by weight of the total composition and azone oil in an amount of approximately 0.25 % to 1 % by weight of the total composition;
wherein the salicylic acid and the azone oil act synergistically as a penetration enhancer to increase skin's permeability of a user for transdermal absorption of the physiologically active ingredients.

4. A personal cleansing composition, the composition comprising:
a caffeine or a methylxanthine selected from theobromine, theophylline, paraxanthine, or pharmaceutically acceptable salts thereof;
physiological active ingredients comprising salicylic acid in an amount of approximately 0.5 % to 2.0 % by weight of the total composition and azone oil in an amount of approximately 0.25 % to 1 % by weight of the total composition;
wherein the salicylic acid and the azone oil act synergistically as a penetration enhancer to increase skin's permeability of a user for transdermal absorption of the physiologically active ingredients.

5. A personal cleansing composition, the composition comprising:
a magnesium salt selected from magnesium sulfate, magnesium chloride, magnesium lactate, magnesium citrate, magnesium aspartate, magnesium gluconate and mixtures thereof; and
physiological active ingredients comprising salicylic acid in an amount of approximately 0.5 % to 2.0 % by weight of the total composition and azone oil in an amount of approximately 0.25 % to 1 % by weight of the total composition;
wherein the salicylic acid and the azone oil act synergistically as a penetration enhancer to increase skin's permeability of a user for transdermal absorption of the physiologically active ingredients.

6. The composition according to any of preceding claims, further comprising magnesium salt in an amount of approximately 0.5 % to 2.0 % by weight of the total composition.

7. The composition according to any of preceding claims, further comprising caffeine in an amount of approximately 0.1 % to 1.0 % by weight of the total composition.

8. The composition according to any of preceding claims, wherein the carnosine and the magnesium salt form a carnosine-magnesium complex on activation with water.

9. The composition according to any of preceding claims, further comprising Epsom salts including naturally containing magnesium sulfate, in addition to other salts.

10. The composition according to any of preceding claims, further comprising at least one essential oil selected from the set of: camphor oil; eucalyptus oil; clove bud oil; and almond oil.

11. The composition as claimed in claim 10, wherein the at least one essential oil is present in an amount of approximately 0.1 % to 2.0 % by weight of the total composition.

12. The composition according to any of preceding claims, further comprising arnica extract, wherein the arnica extract is present in an amount of approximately 0.1 % to 0.5 % by weight of the total composition.

13. The composition according to any of preceding claims, further comprising an exfoliant, wherein the exfoliant is present in an amount of approximately 0.1 % to 0.5 % by weight of the total composition.

14. The composition according to any of preceding claims, wherein the composition form of the water-soluble delivery agent formed as a bath salt or bath bomb or bath melt.

15. The composition according to any of preceding claims, wherein the composition is in the form of a shower gel or bubble bath or soap or water-soluble delivery agent or a bar of soap.
